# EUROPÄISCHE PATENTSCHRIFT

(11) **EP 0 633 038 B1**
(45) Veröffentlichungstag und Bekanntmachung des Hinweises auf die Patenterteilung: **08.07.1998**
(21) Anmeldenummer: 94108064.0
(22) Anmeldetag: 26.05.1994
(51) Int. Cl.: A61M 39/00, F16L 19/00

(54) **Schlauchkupplung**
Tube connector
Raccord de tuyau

(30) Priorität: 01.06.1993 DE 4318101
(43) Veröffentlichungstag der Anmeldung: 11.01.1995
(73) Patentinhaber: STERIMED Medizinprodukte GmbH, 66337 Püttlingen (DE)
(72) Erfinder: Eick, Rüdiger, D-66459 Kirkel (DE); Mehner, Gotthilf, D-66280 Sulzbach (DE)
(74) Vertreter: Rupp, Herbert, Dr.

(56) Entgegenhaltungen:
- EP-A- 0 204 311
- EP-A- 0 415 665
- EP-A- 0 443 868
- CH-A- 670 955
- GB-A- 2 146 405

## Beschreibung

### Technisches Gebiet

Die Erfindung betrifft eine Schlauchkupplung zum Verbinden von Schlauchleitungen für medizinische Zwecke.

### Stand der Technik

Bei den verschiedensten medizinischen Anwendungen ergibt sich die Notwendigkeit, Schlauchenden miteinander lösbar zu verbinden. Für wenig kritische Anwendungen ist es in vielen Fällen ausreichend, die Schlauchenden über ein kurzes, elastisches Schlauchstück zu verbinden, dessen Innendurchmesser etwas geringer ist als der Außendurchmesser der zu verbindenden Schlauchenden. Diese Verbindungstechnik ist jedoch sehr anfällig gegen Zugbelastung und Kontamination.

Eine häufig angewandte Vorgehensweise ist die Verwendung von Luer-Lock-Verbindungen. Diese bestehen aus einem ersten Kupplungsteil mit Innenkonus und außenseitigen Fragmenten eines zweigängigen Außengewindes und einem zweiten Kupplungsteil mit Außenkonus und drehbar aufgesetztem Verbindungsteil mit in Richtung erstes Kupplungsteil gerichtetem Rohrstutzen mit Innengewinde zur Aufnahme des Gewindefragments des ersten Kupplungsteils. Nachdem der Außenkonus in den Innenkonus gesteckt ist, wird die Verbindung der beiden Kupplungsteile durch Zuschrauben des frei drehbaren Rohrstutzen fixiert.

Obwohl diese Verbindungstechnik als bewährt gelten kann, hat es sich in der Praxis gezeigt, daß insbesondere im Zusammenhang mit der Unterdruck-Wunddrainage (Redon-Technik) Probleme auftreten. Beispielsweise können durch Torsion der relativ starrwandigen Vakuumschläuche oder durch unzureichendes Verschrauben der Verbindungselemente Vakuumverluste und damit nachteilige Wirkungen auf die Drainage auftreten. Außerdem erhöht sich bei undichten Verbindungen durch Ansaugen von Keimen die Infektionsgefahr.

### Beschreibung der Erfindung

Die Aufgabe der vorliegenden Erfindung bestand darin, die bekannten Schlauchkupplungen durch eine Arretierung sicherer zu machen ohne deren Handhabung zu erschweren und ohne nennenswerten Mehraufwand bei der Herstellung in Kauf nehmen zu müssen.

Gelöst wird diese Aufgabe dadurch, daß ein Rotationsrastgesperre vorgesehen ist, das gebildet wird durch im mittleren Bereich des ersten Kupplungsteils diametral gegenüberliegende, radial abstehende Verriegelungsleisten, deren Funktionsflächen mit am Innenmantel des elastisch verformbaren Rohrstutzens diametral gegenüberliegend angeordneten Rastvorsprüngen bzw. deren Funktionsflächen zusammenwirken.

Der Gegenstand der Erfindung ergibt sich aus dem Patentanspruch.

Nachstehend wird der Erfindungsgegenstand anhand der Figuren 1 bis 7 näher beschrieben. Es zeigen:
Fig. 1 die Bestandteile einer erfindungsgemäßen Schlauchkupplung in axonometrischer Explosionsdarstellung,
Fig. 2 eine erfindungsgemäße Schlauchkupplung gem. Fig. 1 im vormontierten Zustand,
Fig. 3 eine erfindungsgemäße Schlauchkupplung gem. Fig. 1 in zusammengefügter, verriegelter Situation,
Fig. 4 einen Längsschnitt gem. der Linie IV-IV in Fig. 3,
Fig. 5 einen Querschnitt gem. der Linie V-V in Fig. 4,
Fig. 6 einen Querschnitt gem. der Linie V-V in Fig. 4 in entriegelter Situation
Fig. 7 einen vergrößerten Detailausschnitt aus Fig. 6.

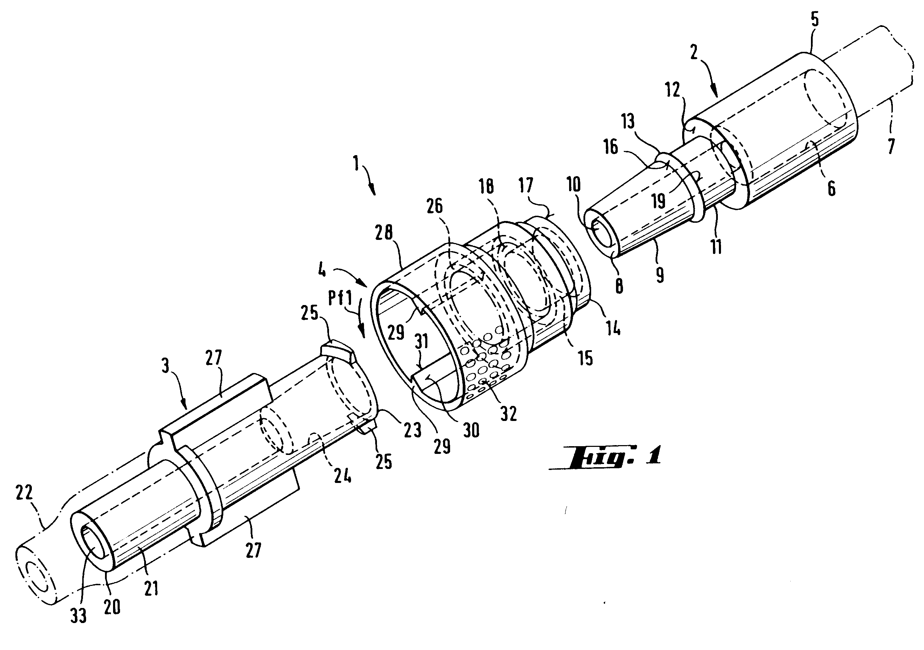

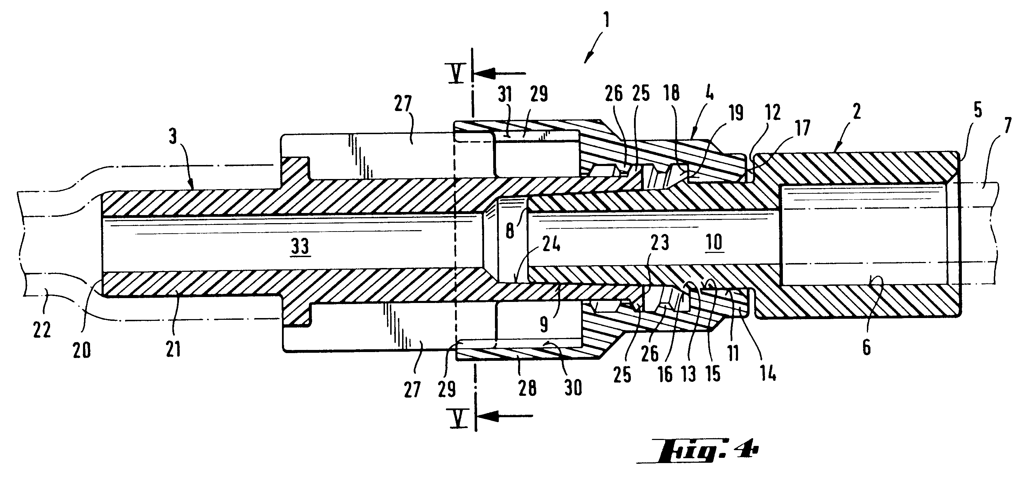

Eine beispielhaft in den Figuren 1 bis 7 wiedergegebene, erfindungsgemäße Schlauchkupplung 1 weist im wesentlichen drei Teile auf, nämlich ein erstes Kupplungsteil 3, ein zweites Kupplungsteil 2, sowie ein Verbindungsteil 4. Das zweite Kupplungsteil 2 ist, wie weiter unten näher beschrieben, mit dem Verbindungsteil 4 verbunden bzw. vormontiert und bildet zusammen mit dem Verbindungsteil 4 einen Teil der Schlauchkupplung. Der andere Teil der Schlauchkupplung wird durch das erste Kupplungsteil 3 gebildet. Das zweite Kupplungsteil 2 weist an seinem schlauchseitigen Ende 5 eine an den Schlauchaußendurchmesser angepaßte Bohrung 6 auf, in welche das eine Schlauchende 7 eingebracht und ggf. in geeigneter Weise fixiert ist.

Das der Schlauchaufnahmebohrung 6 gegenüberliegende Ende 8 des zweiten Kupplungsteils 2 ist als Außenkonus 9, welcher die Dichtfläche des zweiten Kupplungsteils 2 bildet, ausgebildet. Das zweite Kupplungsteil 2 ist von einer zentralen Bohrung 10 durchsetzt.

Zwischen dem Schlauchaufnahmeende 5 und dem Konus 9 des zweiten Kupplungsteils 2 ist ein zylindrischer Bereich 11 in Art eines Lagerzapfens vorgesehen, welcher axial am schlauchseitigen Ende 5 durch eine Schulter 12 und am konusseitigen Ende 8 durch einen Ringwulst 13 begrenzt ist. Der Durchmesser des Lagerzapfens 11 entspricht im wesentlichen dem großen Konusdurchmesser.

Das Verbindungsteil 4 weist an seinem dem zweiten Kupplungsteil 2 zugewandten Ende 14 eine Lagerbohrung 15 auf, welche im Durchmesser und der axialen Länge auf den Lagerzapfen 11 abgestimmt ist.

Unter elastischer Verformung der entsprechenden Bereiche, begünstigt durch die schräg ansteigende Aufgleitfläche 16 des Ringwulstes 13, kann das Verbindungsteil 4 auf das zweite Kupplungsteil 2 aufgeschoben bzw. aufgeschnappt werden.

Senkrecht zur Längsachse stehende Schulterflächen 17, 18 bzw. 12, 19 des Verbindungsteiles 4 und des zweiten Kupplungsteils 2 bilden die axiale formschlüssige Fixierung des Verbindungsteiles 4 auf dem zweiten Kupplungsteil 2 unter Beibehaltung der Drehbarkeit gegeneinander.

Das erste Kupplungsteil 3 weist an seinem dem zweiten Kupplungsteil 2 abgewandten Ende 20 einen Tubus 21 zur Aufnahme des anderen Schlauchendes 22 auf. Das erste Kupplungsteil 3 wird von einer zentralen Bohrung 33 durchsetzt. Der Außendurchmesser des Tubus 21 ist an den Innendurchmesser des Schlauches 22 angepaßt. In das dem zweiten Kupplungsteil 2 zugewandten Ende 23 des ersten Kupplungsteils 3 ist ein Innenkonus 24 eingelassen, welcher mit dem Außenkonus 9 des zweiten Kupplungsteils 2 korrespondiert und die Dichtfläche des anderen Kupplungsteils bildet. An dem dem einen Kupplungsteil zugewandten Ende 23 des ersten Kupplungsteils 3 sind diametral gegenüberliegende Vorsprünge 25 außen aufgebracht, welche Fragmente eines zweigängigen Außengewindes darstellen. Diese Vorsprünge 25 greifen in ein entsprechendes Innengewinde 26 des Verbindungsteils 4 ein, wodurch ein axiales Annähern bzw. Kontaktieren der Dichtflächen 9, 24 beim entsprechenden Verdrehen der Kupplungsteile bzw. des Verbindungsteils 4 gegeben ist.

Im mittleren Bereich des ersten Kupplungsteils 3 sind diametral gegenüberliegend, radial abstehende Verriegelungsleisten 27 angeformt, welche Funktionsflächen eines Rotationsrastgesperres bilden.

Das Verbindungsteil 4 weist einen in Richtung des ersten Kupplungsteils 3 abstehenden Rohrstutzen 28 auf, dessen Innendurchmesser entsprechend der radialen Erstreckung der Verriegelungsleisten 27 des ersten Kupplungsteils 3 angepaßt ist.

Am Innenmantel des Rohrstutzens 28 sind diametral gegenüberliegend Rast vorsprünge 29 angeordnet, welche mit den Verriegelungsleisten 27 resp. mit dessen Funktionsflächen zusammenwirkende Gegenfunktionsflächen ausbilden.

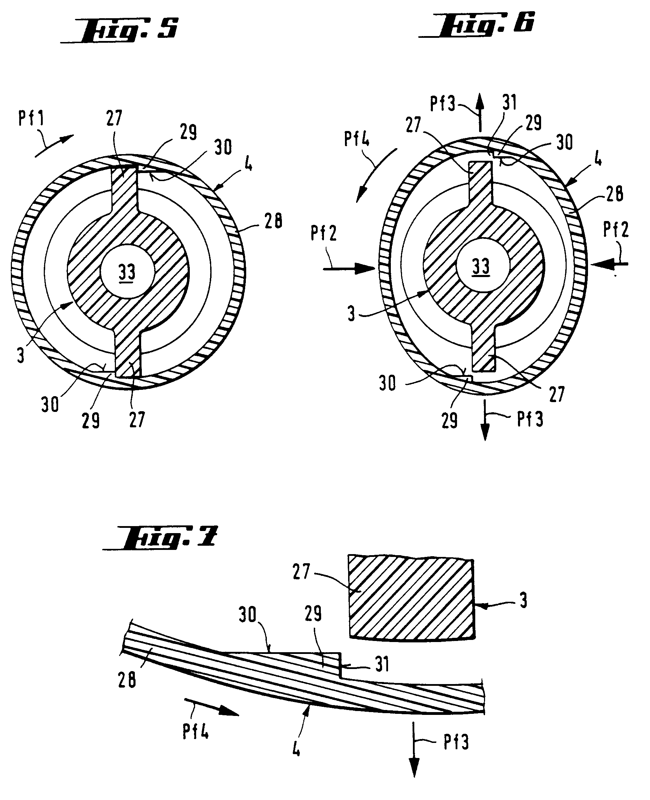

Diese Rastvorsprünge 29 weisen eine Aufgleitfläche 30 und eine Rastfläche 31 auf (Fig. 7), derart, daß im Zusammenwirken mit den Verriegelungsleisten 27 die Drehung des Verbindungsteils 4 gegenüber dem ersten Kupplungsteil 3 nur in einer Richtung (Pf 1) möglich ist, nämlich in jener, bei welcher sich aufgrund der Gewindeverbindung 25, 26 die Dichtflächen 9, 24 annähern.

Aufgrund der Elastizität der Wandung des Rohrstutzens 28 können die Vorsprünge unter Einwirkung der Verriegelungsleisten 27 radial nach außen ausweichen.

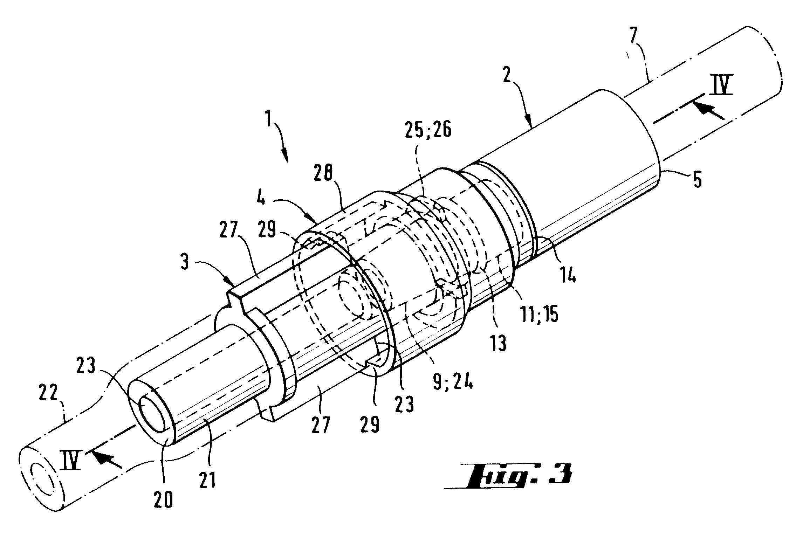

Die Gewindegänge im Verbindungsteil 4 und an dem ersten Kupplungsteil 3, die Anordnung der Rastvorsprünge 29 im Verbindungsteil 4 und die Verriegelungsleisten 27 an dem ersten Kupplungsteil 3 sind derart aufeinander abgestimmt, daß bei absolut dichtend zusammengeführten konischen Dichtflächen 9, 24 der Kupplungsteile ein Aufdrehen (Pf 4) resp. ein Auseinanderführen der Dichtflächen durch das Gesperre gern. Fig. 3, 4 und 5 verhindert bzw. blockiert wird.

Zum Lösen der Verbindung müssen die Rastvorsprünge 29 des Verbindungsteils 4 radial nach außen bewegt, d.h. außer Eingriff mit den Verriegelungsleisten 27 der 1. Kupplungsteils 3 gebracht werden.

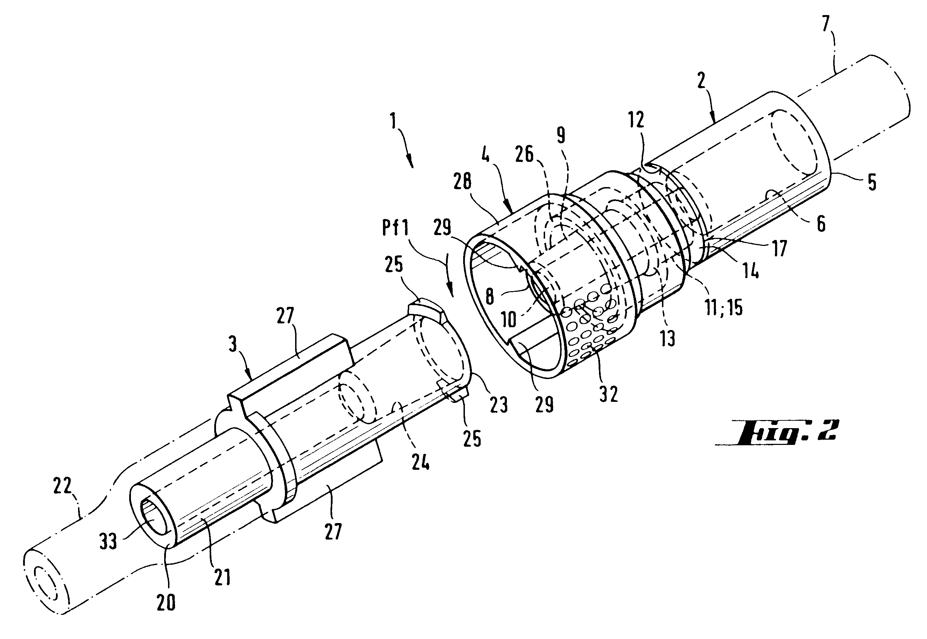

Durch gleichzeitige Krafteinwirkung auf den Rohrstutzen 28 in Richtung der Pfeile (Pf 2) resp. der daraus resultierenden Verformung (Pf 3) (Fig. 6) kann die Verrastung aufgehoben werden, wonach die Kupplungsteile auseinandergeschraubt werden können. Die Krafteinleitung erfolgt an diametral gegenüberliegenden Punkten des Rohrstutzens 28, 90 Grad versetzt zu den Rastvorsprüngen 29. Diese Krafteinleitzonen können durch eine Strukturierung 32 (Fig. 1 + 2) in der Griffigkeit verbessert und kenntlich gemacht werden.

## Patentansprüche

1. Schlauchkupplung zum Verbinden von Schlauchleitungen für medizinische Zwecke umfassend ein erstes Kupplungsteil (3) mit Innenkonus (24) und außenseitigen diametral gegenüberliegenden Vorsprüngen (25), welche ein Fragment eines zweigängigen Außengewindes darstellen und ein zweites Kupplungsteil (2) mit Außenkonus (9) und frei drehbarem Verbindungsteil (4), dessen in Richtung erstes Kupplungsteil (3) offener Rohrstutzen (28) ein Innengewinde (26) zur Aufnahme des durch die Vorsprünge (25) gebildeten Außengewindes aufweist, dadurch gekennzeichnet, daß ein Rotationsrastgesperre vorgesehen ist, das gebildet wird durch im mittleren Bereich des ersten Kupplungsteils (3) diametral gegenüberliegende, radial abstehende Verriegelungsleisten (27), deren Funktionsflächen mit am Innenmantel des elastisch verformbaren Rohrstutzens (28) diametral gegenüberliegend angeordneten Rastvorsprüngen (29) bzw. deren Funktionsflächen (30,31) zusammenwirken.

## Claims

1. Tube coupling for the connection of tube lines for medical purposes, comprising a first coupling part (3) with an inner cone (24) and diametrically opposite projections (25) on the outside, which represent a fragment of a two-turn external thread, and a second coupling part (2) with an outer cone (9) and a freely rotatable connection part (4) whose pipe connector (28) which is open in the direction of a first coupling part (3) has an internal thread (26) to receive the external thread formed by the projections (25), characterized in that a rotational latching mechanism is provided, which is formed by radially projecting locking webs (27) which are located diametrically opposite one another in the central region of the first coupling part (3) and whose functional surfaces interact with latching projections (29) or their functional surfaces (30, 31) located diametrically opposite one another on the inner surface of the elastically deformable pipe connector (28).

## Revendications

1. Raccord pour tube pour connecter des tubes souples dans des applications médicales, comprenant une première partie de raccord (3) avec un cône intérieur (24) et des saillies extérieures (25) diamétralement opposées qui constituent un morceau d'un filetage extérieur à deux pas ainsi qu'une deuxième partie de raccord (2) avec un cône extérieur (9) et une pièce de liaison (4) tournant librement, dont le tronçon tubulaire (28), ouvert en direction de la première partie de raccord (3), présente un filetage intérieur (26) destiné à recevoir le filetage extérieur formé par les saillies (25), caractérisé par le fait qu'il est prévu un moyen de verrouillage en rotation à crans, qui est formé par des nervures de verrouillage (27) radiales, diamétralement opposées, dans la partie médiane de la première partie de raccord (3), dont les surfaces actives coopèrent avec des saillies formant crans (29) ou les surfaces actives (30, 31) de ces dernières, diamétralement opposées, sur la paroi intérieure du tronçon de tube (28) déformable élastiquement.
